# EUROPEAN PATENT APPLICATION

(11) **EP 4 328 209 A1**
(43) Date of publication of application: **28.02.2024**
(21) Application number: 21943873.6
(22) Date of filing: 29.11.2021
(51) Int. Cl.: C04B 38/06, C04B 35/16, C04B 35/622, A24F 40/70, A61M 11/00

(54) **POROUS ATOMIZING CORE CAPABLE OF RELEASING NEGATIVE IONS AND PREPARATION METHOD THEREFOR**

(30) Priority: 04.06.2021 CN 202110627482
(71) Applicant: Shenzhen Huachengda Precision Industry Co., Ltd., Shenzhen, Guangdong 518000 (CN)
(72) Inventor: CHEN, Ping, Shenzhen, Guangdong 518000 (CN)
(74) Representative: Westphal, Mussgnug & Partner, Patentanwälte mbB
(86) International application number: PCT/CN2021/133938
(87) International publication number: WO 2022/252521

(57) **Abstract**

The present invention provides a porous atomizing core capable of releasing negative ions and method for preparing same. The porous atomizing core includes raw materials in parts by mass as follows: 50-90 parts by mass of tourmaline, 0-30 parts by mass of ceramic powder, 10-30 parts by mass of pore-forming agent, 0-30 parts by mass of sintering aid, 0-35 parts by mass of paraffin, and 0-2 parts by mass of surfactant. The ceramic powder includes at least one of negative ion powder, far infrared powder, attapulgite clay, feldspar and zeolite. In the porous atomizing core capable of releasing negative ions of the present invention, tourmaline is used as one of the raw materials, utilizing its characteristics of pyroelectricity, piezoelectricity, infrared radiation, release of negative oxygen ions and bioelectricity, to decompose and remove harmful gases in the atomized liquid and air, so that the porous atomizing core is environmentally friendly and has good atomizing effect, which satisfies the people's pursuit of healthy products, and simultaneously improves the service life of the atomizing core.

## Description

### FIELD

The invention relates to the technical field of atomization, in particular to a porous atomizing core capable of releasing negative ions and a method for preparing the porous atomizing core.

### BACKGROUND

With the continuous development of the atomization industry, the use of porous atomizing core has gradually become a part of people's lives, but because of the diversity of atomized liquid in different fields of use, ordinary atomizing core is not guaranteed to be able to atomize for a long period of time, and the expiration of the atomized liquid leads to the growth of bacteria, which has also greatly reduced the service life of the atomizer, especially in the electronic cigarette atomization and medical atomization industry. Moreover, with the constant pursuit of health concept, it is particularly important to research a healthy and environmentally friendly atomizing core.

At present, one of the solutions is to choose environmentally friendly materials and to replace the atomizing core regularly, which not only greatly limits the use of the atomizing core, but also increases the use cost.

### SUMMARY

The technical issue to be addressed by the invention is to provide a porous atomizing core capable of releasing negative ions and a method for preparing the porous atomizing core.

The technical solution adopted by the invention to solve the above technical issue is to provide a porous atomizing core capable of releasing negative ions including raw materials in parts by mass as follows: 50-90 parts by mass of tourmaline, 0-30 parts by mass of ceramic powder, 10-30 parts by mass of pore-forming agent, 0-30 parts by mass of sintering aid, 0-35 parts by mass of paraffin, and 0-2 parts by mass of surfactant;

Wherein the ceramic powder includes at least one of negative ion powder, far infrared powder, attapulgite clay, feldspar and zeolite.

Preferably, the tourmaline is a primary modified tourmaline.

Preferably, the primary modified tourmaline is obtained by the following modification method: adding 100 parts by mass of tourmaline into hydrated cerium nitrate solution with a mass fraction of 1% -10%, placing it in a chromatography column for cyclic overloading for 2-5h, and then magnetically stirring the overloaded suspension, slowly dropping aqueous ammonia with a mass fraction of 5% -10% and stopping dropping when the pH value is 10, then adding pure water and stirring and washing several times, filtering off clear liquid, drying in an oven at a temperature of 70 °C -90 °C, and then placing it in a sintering furnace and calcining at a temperature of 500 °C-600 °C for 4-6h.

Preferably, the tourmaline is a secondary modified tourmaline.

Preferably, the secondary modified tourmaline is obtained by the following modification method: applying 100 parts by mass of primary modified tourmaline and 200 parts by mass of absolute ethanol, mixing them and magnetically stirring, and then adding 60 parts by mass of butyl titanate, stirring for 2-3h and filtering off clear liquid after stirring, drying in an oven at a temperature of 70 °C -90 °C, and placing it in a sintering furnace and calcining at a temperature of 450 °C-550 °C for 3-4h.

Preferably, the primary modified tourmaline is obtained by the following modification method: adding 100 parts by mass of tourmaline into hydrated cerium nitrate solution with a mass fraction of 1% -10%, placing it in a chromatography column for cyclic overloading for 2-5h, and then magnetically stirring the overloaded suspension, slowly dropping aqueous ammonia with a mass fraction of 5% -10% and stopping dropping when the pH value is 10, then adding pure water and stirring and washing several times, filtering off clear liquid, drying in an oven at a temperature of 70 °C -90 °C, and then placing it in a sintering furnace and calcining at a temperature of 500 °C-600 °C for 4-6h.

Preferably, the tourmaline has a particle size of 100 mesh-1500 mesh;

The ceramic powder has a particle size of 100 mesh-1500 mesh.

Preferably, the sintering aid is at least one of metal oxide and glass powder, with a particle size of 325 mesh- 2000 mesh, and an initial melting temperature of 300°C-500°C;

The pore-forming agent is at least one of wheat flour, PS microspheres, carbon powder and grain husk powder, with a particle size of 80 mesh-1000 mesh.

Preferably, the paraffin is a semi-refined or refined paraffin with a melting point of 40°C-100°C.

Preferably, the surfactant is at least one of dehydrated sorbitol fatty acid ester, polysorbate, stearic acid and oleic acid.

A method for preparing a porous atomizing core capable of releasing negative ions including the following steps:

S1, mixing the raw materials in parts by mass, and pressing to form a blank;

S2, placing the blank into a sintering furnace and heating up to 600°C-800°C at a heating rate of 1°C/min -10°C/min for sintering.

The invention has the following beneficial effects: tourmaline is used as one of the raw materials, utilizing its characteristics of pyroelectricity, piezoelectricity, infrared radiation, release of negative oxygen ions and bioelectricity, to decompose and remove harmful gases in the atomized liquid and air, so that the porous atomizing core is environmentally friendly and has good atomizing effect, which satisfies the people's pursuit of healthy products, and simultaneously improves the service life of the atomizing core.

### BRIEF DESCRIPTION OF THE DRAWINGS

The present invention will be further described in the following, in conjunction with the accompanying drawings and embodiments. In the drawings:
FIG. 1 is a SEM image of the secondary modified tourmaline of the present invention;
FIG. 2 is a SEM image of the porous atomizing core capable of releasing negative ions of the present invention.

### DETAILED DESCRIPTION

For a better understanding of the technical features, purposes and effects of the invention, the specific implementations of the invention are described in detail with reference to the accompanying drawings.

The porous atomizing core capable of releasing negative ions of the present invention includes raw materials in parts by mass as follows: 50-90 parts by mass of tourmaline, 0-30 parts by mass of ceramic powder, 10-30 parts by mass of pore-forming agent, 0-30 parts by mass of sintering aid, 0-35 parts by mass of paraffin, and 0-2 parts by mass of surfactant.

Tourmaline is a kind of silicate mineral with annular structure. Due to its special crystal structure, it has permanent spontaneous polarization effect, giving it characteristics of pyroelectricity, piezoelectricity, infrared radiation, release of negative oxygen ions and bioelectricity. The far infrared produced by tourmaline can kill the bacteria in the atomized liquid, promote blood circulation and metabolism. The negative ions produced by tourmaline can ionize the atomized liquid, making it weak alkaline, which is more conducive to physical and mental health. The micro-current produced by tourmaline is consistent with the human body's biological micro-current, which can cause the phenomenon of bioelectricity in the human body.

The tourmaline has a preferred particle size of 100 mesh-1500 mesh.

Further, in order to improve the negative ion and far infrared release properties of the tourmaline, the tourmaline is modified to form primary modified tourmaline or secondary modified tourmaline.

The primary modified tourmaline is obtained by the following modification method: adding 100 parts by mass of tourmaline into hydrated cerium nitrate solution with a mass fraction of 1% -10%, mixing them and placing the mixture in a chromatography column for cyclic overloading for 2-5h, and then magnetically stirring the overloaded suspension, slowly dropping aqueous ammonia with a mass fraction of 5% -10% and stopping dropping when the pH value is 10, then adding pure water and stirring and washing several times (e.g. twice), filtering off clear liquid, drying in an oven at a temperature of 70 °C -90 °C, and then placing it in a sintering furnace and calcining at a temperature of 500 °C-600 °C for 4-6h, the product obtained after calcination is primary modified tourmaline.

The above primary modified tourmaline is modified again to obtain secondary modified tourmaline and the modification method is as follows: applying 100 parts by mass of primary modified tourmaline and 200 parts by mass of absolute ethanol, mixing them and magnetically stirring, and then adding 60 parts by mass of butyl titanate, stirring for 2-3h and filtering off clear liquid after stirring, drying in an oven at a temperature of 70 °C -90 °C, and placing it in a sintering furnace and calcining at a temperature of 450 °C-550 °C for 3-4h, the product obtained after calcination is secondary modified tourmaline.

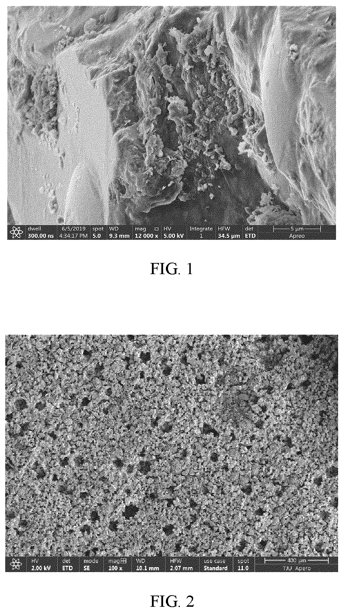

The SEM image of the secondary modified tourmaline is shown in Fig. 1, from which it can be seen that the surface of the secondary modified tourmaline is smooth. Tourmaline exhibits many tiny particles on the surface after being compounded with TiO₂, which indicates a good modification result.

In the primary modification, there are two variable valence states of Ce (cerium), Ce³⁺ can be transformed into Ce⁴⁺ under the condition of light. There is electron-hole recombination in the transformation process, and the existence of electrostatic field at both ends of the tourmaline will make the electrons involved in the recombination migrate under the action of the electric field, which reduces the probability of of electron-hole recombination, making the surface of the tourmaline/Ce composite material accumulates a large amount of energy, thus lead to an increase in the release of negative oxygen ions. In the secondary modification, the generated titanium dioxide has a photocatalytic effect, which enhances the effect of the primary modification, and in the light or ultraviolet irradiation, the electrons on the surface of the TiO₂ absorb enough energy to detach, thus promoting the release effect of negative ions and far infrared of tourmaline.

The ceramic powder includes at least one of negative ion powder, far infrared powder, attapulgite clay, feldspar and zeolite, with a particle size of 100 mesh-1500 mesh. The feldspar is potassium feldspar and/or albite.

The sintering aid is at least one of metal oxide and glass powder, with a particle size of 325 mesh- 2000 mesh, and an initial melting temperature of 300°C-500°C. The metal oxide may be at least one of magnesium oxide, titanium dioxide and niobium oxide.

The pore-forming agent is at least one of wheat flour, PS microspheres, carbon powder and grain husk powder, with a particle size of 80 mesh-1000 mesh.

The paraffin is a semi-refined or refined paraffin with a melting point of 40°C-100°C.

The surfactant is at least one of dehydrated sorbitol fatty acid ester (Span), polysorbate (Tween), stearic acid and oleic acid.

The method for preparing a porous atomizing core capable of releasing negative ions of the present invention includes the following steps:
S1, mixing the raw materials in parts by mass, and pressing to form a blank.

For press molding, either hot pressing or dry pressing may be used.

Furthermore, for hot pressing molding, of the raw materials, sintering aid is preferably 10-30 parts by mass, paraffin is preferably 15-35 parts by mass, and surfactant is preferably 0.05-2 parts by mass.

For dry pressing molding, sintering aid, paraffin and surfactant may not be added to the raw material.

S2, placing the blank into a sintering furnace and heating up to 600°C-800°C at a heating rate of 1°C/min -10°C/min for sintering to obtain a porous atomizing core by primary sintering.

It can be understood that the method of preparing the porous atomizing core capable of releasing negative ions of the present invention is not limited to hot pressing or dry pressing as described above, and other methods of preparing the atomizing core by molding may be used.

The SEM image of the porous atomizing core capable of releasing negative ions of the present invention is shown in Fig. 2, from which it can be seen that the atomizing core has many pores and uniform pores, and the uniform pores can sufficiently ensure the function effect of tourmaline and make atomization effect better.

The present invention will be further described below by way of specific embodiments.

### Embodiment 1

The raw materials in parts by mass are as follows: 80 parts by mass of non-modified tourmaline, 20 parts by mass of glass powder, 20 parts by mass of wheat flour, 20 parts by mass of paraffin, 0.5 parts by mass of oleic acid. The above raw materials are mixed and subjected to perform hot pressing, and are sintered once at 600°C-800°C to form a porous atomizing core.

### Embodiment 2

Tourmaline is the primary modified tourmaline, and the rest is the same as in Embodiment 1.

### Embodiment 3

Tourmaline is the secondary modified tourmaline, and the rest is the same as in Embodiment 1.

### Embodiment 4

The raw materials in parts by mass are as follows: 80 parts by mass of non-modified tourmaline, 20 parts by mass of glass powder, 5 parts by mass of negative ion powder, 20 parts by mass of wheat flour, 20 parts by mass of paraffin, 0.5 parts by mass of oleic acid. The above raw materials are mixed and subjected to perform hot pressing, and are sintered once at 600°C-800°C to form a porous atomizing core.

### Embodiment 5

The raw materials in parts by mass are as follows: 80 parts by mass of secondary modified tourmaline, 5 parts by mass of feldspar, 15 parts by mass of attapulgite clay, 20 parts by mass of wheat flour. The above raw materials are sintered once at 600°C-800°C to form a porous atomizing core.

### Comparative Embodiment 1

Tourmaline in Embodiment 1 is replaced by aluminium oxide, and the rest is the same as in Embodiment 1.

The atomizing core obtained in Embodiments 1-5 and Comparative Embodiment 1 were tested about property of releasing negative ions: the COM-3010PRO Solid Negative Ion Tester and COM-3200PRO II Air Negative Ion Tester were used to test the content of solid and air negative ions, and the air negative ions were tested for 20-60 min in enclosed space of 1 m³. The size of the atomizing core sample is 1cm in thickness and 10cm in diameter. The test results are shown in Table 1 below.

**Table 1**

| Embodiments | Solid negative ions (pcs/cm³ ) | Air negative ions (pcs/cm³ ) |
|---|---|---|
| Embodiment 1 | 147 | 660-710 |
| Embodiment 2 | 430 | 1680-1750 |
| Embodiment 3 | 520 | 4690-4760 |
| Embodiment 4 | 500 | 3360-3530 |
| Embodiment 5 | 520 | 4520-4630 |
| Comparative Embodiment 1 | 10 | 60-70 |

As can be seen from Table 1, the addition of tourmaline in the present invention makes the atomizing core have functions of releasing negative ions and so on, and the modified tourmaline is more effective; the addition of negative ion powder also has the same effect.

The above are only embodiments of the present invention and do not limit the scope of the present invention. Any equivalent structure or equivalent process transformation made based on the content of the specification and the accompanying drawings of the present invention, or direct or indirect application in other related technical fields, fall equally within the patent scope of the present invention.

## Claims

1. A porous atomizing core capable of releasing negative ions, **characterized in** comprising raw materials in parts by mass as follows: 50-90 parts by mass of tourmaline, 0-30 parts by mass of ceramic powder, 10-30 parts by mass of pore-forming agent, 0-30 parts by mass of sintering aid, 0-35 parts by mass of paraffin, and 0-2 parts by mass of surfactant;
wherein the ceramic powder comprises at least one of negative ion powder, far infrared powder, attapulgite clay, feldspar and zeolite.

2. The porous atomizing core capable of releasing negative ions according to claim 1, **characterized in that** the tourmaline is a primary modified tourmaline.

3. The porous atomizing core capable of releasing negative ions according to claim 2, **characterized in that** the primary modified tourmaline is obtained by the following modification method: adding 100 parts by mass of tourmaline into hydrated cerium nitrate solution with a mass fraction of 1% -10%, placing it in a chromatography column for cyclic overloading for 2-5h, and then magnetically stirring the overloaded suspension, slowly dropping aqueous ammonia with a mass fraction of 5% -10% and stopping dropping when the pH value is 10, then adding pure water and stirring and washing several times, filtering off clear liquid, drying in an oven at a temperature of 70 °C -90 °C, and then placing it in a sintering furnace and calcining at a temperature of 500 °C-600 °C for 4-6h.

4. The porous atomizing core capable of releasing negative ions according to claim 1, **characterized in that** the tourmaline is a secondary modified tourmaline.

5. The porous atomizing core capable of releasing negative ions according to claim 4, **characterized in that** the secondary modified tourmaline is obtained by the following modification method: applying 100 parts by mass of primary modified tourmaline and 200 parts by mass of absolute ethanol, mixing them and magnetically stirring, and then adding 60 parts by mass of butyl titanate, stirring for 2-3h and filtering off clear liquid after stirring, drying in an oven at a temperature of 70 °C -90 °C, and placing it in a sintering furnace and calcining at a temperature of 450 °C-550 °C for 3-4h.

6. The porous atomizing core capable of releasing negative ions according to claim 5, **characterized in that** the primary modified tourmaline is obtained by the following modification method: adding 100 parts by mass of tourmaline into hydrated cerium nitrate solution with a mass fraction of 1% -10%, placing it in a chromatography column for cyclic overloading for 2-5h, and then magnetically stirring the overloaded suspension, slowly dropping aqueous ammonia with a mass fraction of 5% -10% and stopping dropping when the pH value is 10, then adding pure water and stirring and washing several times, filtering off clear liquid, drying in an oven at a temperature of 70 °C -90 °C, and then placing it in a sintering furnace and calcining at a temperature of 500 °C-600 °C for 4-6h.

7. The porous atomizing core capable of releasing negative ions according to any one of claims 1-6, **characterized in that** the tourmaline has a particle size of 100 mesh-1500 mesh;
the ceramic powder has a particle size of 100 mesh-1500 mesh.

8. The porous atomizing core capable of releasing negative ions according to any one of claims 1-6, **characterized in that** the sintering aid is at least one of metal oxide and glass powder, with a particle size of 325 mesh- 2000 mesh, and an initial melting temperature of 300°C-500°C;
the pore-forming agent is at least one of wheat flour, PS microspheres, carbon powder and grain husk powder, with a particle size of 80 mesh-1000 mesh.

9. The porous atomizing core capable of releasing negative ions according to any one of claims 1-6, **characterized in that** the paraffin is a semi-refined or refined paraffin with a melting point of 40°C-100°C;
the surfactant is at least one of dehydrated sorbitol fatty acid ester, polysorbate, stearic acid and oleic acid.

10. A method for preparing a porous atomizing core capable of releasing negative ions according to any one of claims 1-9, **characterized in** comprising the following steps:
S1, mixing the raw materials in parts by mass, and pressing to form a blank;
S2, placing the blank into a sintering furnace and heating up to 600°C-800°C at a heating rate of 1°C/min -10°C/min for sintering.
